# EUROPÄISCHE PATENTSCHRIFT

(11) **EP 1 565 740 B1**
(45) Veröffentlichungstag und Bekanntmachung des Hinweises auf die Patenterteilung: **08.10.2008**
(21) Anmeldenummer: 03785548.3
(22) Anmeldetag: 28.11.2003
(51) Int. Cl.: G01N 33/00, B60H 3/00

(54) **VERFAHREN ZUR ERFASSUNG VON GERÜCHEN**
METHOD FOR DETECTING ODOURS
PROCEDE POUR RECONNAITRE DES ODEURS

(30) Priorität: 29.11.2002 DE 10255705
(43) Veröffentlichungstag der Anmeldung: 24.08.2005
(73) Patentinhaber: paragon AG, 33129 Delbrück (DE)
(72) Erfinder: INGRISCH, Kurt, 72762 Reutlingen (DE); BAUER, Michael, 72076 Tuebingen (DE); KRUMMEL, Christian, 72138 Kirchentellinsfurt (DE)
(74) Vertreter: Lelgemann, Karl-Heinz
(86) Internationale Anmeldenummer: PCT/DE2003/003953
(87) Internationale Veröffentlichungsnummer: WO 2004/051262

(56) Entgegenhaltungen:
- WO-A-00/25108
- WO-A2-96/35115
- US-B1- 6 360 584
- US-B2- 6 484 559
- GUI-ZHEN YAN ET AL: "The Investigation of an Integrated Gas Sensor Array" FIRST IEEE INTERNATIONAL CONFERENCE ON SENSORS, 12. Juni 2002 (2002-06-12), XP002277423 Orlando, USA
- FERRARI V ET AL: "Multisensor array of mass microbalances for chemical detection based on resonant piezo-layers of screen-printed PZT" SENSORS AND ACTUATORS B, ELSEVIER SEQUOIA S.A., LAUSANNE, CH, Bd. 68, Nr. 1-3, 25. August 2000 (2000-08-25), Seiten 81-87, XP004216596 ISSN: 0925-4005
- PATENT ABSTRACTS OF JAPAN vol. 012, no. 182 (M-702), 27. Mai 1988 (1988-05-27) & JP 62 292521 A (MITSUBISHI ELECTRIC CORP), 19. Dezember 1987 (1987-12-19)

## Beschreibung

Die Erfindung betrifft ein Verfahren nach dem Oberbegriff des Patentanspruchs 1.

Die WO96/35115 A zeigt ein Verfahren zur Detektion von Diesel- und Benzinabgasen und zur Steuerung einer Umluftklappe eines Kraftfahrzeugs anhand des erfassten Gehalts der Dieselabgase bzw. der Benzinabgase in der Umgebungsluft. Hierbei werden mehrere Sensoren eingesetzt, mittels denen unterschiedliche in Diesel- und Benzinabgasen enthaltene Gaskomponenten erfasst werden. Hierbei handelt es sich insbesondere um CO, Kohlenwasserstoffe, NOx, SO₂ sowie um in Dieselabgasen enthaltene Geruchsstoffe. Mittels der erfassten Sensorsignale kann der Diesel- bzw. der Benzinabgasgehalt der ein Kraftfahrzeug umgebenden Luft erfasst werden, wobei dann eine Umluftklappe der Kraftfahrzeugklimatisierungsanlage in Abhängigkeit von dem Dieselabgas- bzw. Benzinabgasgehalt der Umgebungsluft gesteuert werden kann.

Der Erfindung liegt - ausgehend von dem eingangs geschilderten Stand der Technik - die Aufgabe zugrunde, ein Verfahren zur Erfassung von Geruch zur Verfügung zu stellen, mittels dem unterschiedliche Gerüche sicher und einfach erkannt sowie von anderen Gerüchen oder geruchfreien Gasmischungen unterschieden werden können.

Diese Aufgabe wird erfindungsgemäß durch die Merkmale im kennzeichnenden Teil des Patentanspruchs 1 gelöst.

Das erfindungsgemäße Verfahren zur Erfassung von Gerüchen bietet den Vorteil, dass dadurch Gerüche sicher und einfach erkannt sowie von anderen Gerüchen oder geruchfreien Gasmischungen unterschieden werden können.

Vorteilhafte Weiterbildungen der Erfindung ergeben sich aus den in den abhängigen Patentansprüchen 2 bis 10 angegebenen Merkmalen.

Im Folgenden wird die Erfindung anhand von fünf Figuren weiter erläutert.
- Figur 1: zeigt beispielhaft die Verknüpfung zwischen Gerüchen, Gaskomponenten und Sensoren, wie sie bei dem erfindungsgemäßen Verfahren zum Einsatz kommen kann.
- Figur 2: zeigt den Aufbau einer möglichen Ausführungsform eines Halbleiter-Gassensors im Querschnitt.
- Figur 3: zeigt den Aufbau des Gassensors gemäß Figur 2 in der Draufsicht.
- Figur 4: zeigt den Aufbau des Gassensors gemäß Figur 2 in der Draufsicht mit einer gassensitiven Schicht.
- Figur 5: zeigt in Form eines Blockdiagramms den prinzipiellen Aufbau eines Sensorsystems für das erfindungsgemäße Verfahren.

### Wege zur Ausführung der Erfindung

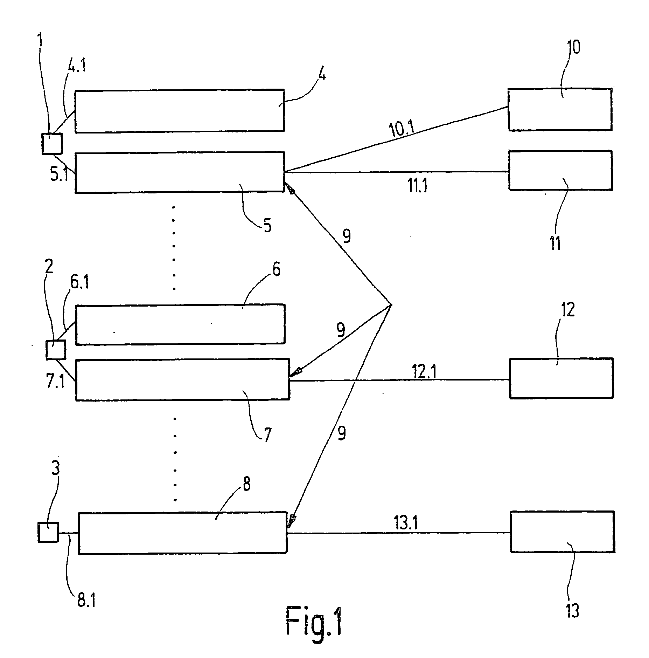

Bei dem erfindungsgemäßen Verfahren zur Erfassung von Gerüchen werden, wie in Figur 1 gezeigt ist, Sensoren 10 bis 13 zur Erfassung von Gasen mit den Gaskomponenten 4 bis 8 verknüpft, zu deren Erfassung sie verwendet werden sollen. Die Gaskomponenten 4 bis 8 wiederum werden mit den entsprechenden Gerüchen 1 bis 3 verknüpft.

So ist, wie in Figur 1 gezeigt ist, der Gassensor 10 über die Verknüpfung 10.1 mit der Gaskomponente 5 und der Gassensor 11 über die Verknüpfung 11.1 ebenfalls mit der Gaskomponente 5 verknüpft. Wird eine Veränderung des Widerstands der Sensoren 10 und 11 festgestellt, bedeutet dies, dass eine Veränderung in der Konzentration der Gaskomponente 5 stattgefunden hat. Verändert sich der Widerstand des Sensors 12 oder des Sensors 13, so wird, da der Sensor 12 und der Sensor 13 nicht mit der Gaskomponente 5 verknüpft sind, nicht auf eine Veränderung der Konzentration der Gaskomponente 5 geschlossen. Der Sensor 12 ist über die Verknüpfung 12.1 mit der Gaskomponente 7 verknüpft. Verändert sich der Widerstand des Sensors 12, wird dies daher als eine Veränderung der Konzentration der Gaskomponente 7 interpretiert. Der Sensor 13 schließlich ist über die Verknüpfung 13.1 mit der Gaskomponente 8 verknüpft. Ändert sich der Widerstand des Sensors 13, bedeutet dies, dass sich die Konzentration der Gaskomponente 8 verändert hat.

Der zu erfassende Geruch 1 setzt sich aus den beiden Gaskomponenten 4 und 5 zusammen, was durch die beiden Verknüpfungen 4.1 bzw. 5.1 veranschaulicht wird. Da es sich bei der Gaskomponente 5 um ein Leitgas handelt, was in Figur 1 durch eine dickere Linie angedeutet und durch die Pfeile 9 zusätzlich gekennzeichnet ist, genügt es, eine Veränderung der Gaskomponente 5 zu erfassen, um auf das Vorhandensein des Geruchs 1 schließen zu können. Eine Erfassung der Gaskomponente 4 ist daher nicht erforderlich, was jedoch ohne weiteres durch einen zusätzlichen nicht gezeigten Sensor möglich wäre. Eine Veränderung in der Konzentration des Geruchs 1 wird somit mit Hilfe der beiden Sensoren 10 und 11 erkannt. Für die Erfassung des Geruchs 2 gilt prinzipiell das Gleiche. Der Geruch 2 setzt sich aus den beiden Gaskomponenten 6 und 7 zusammen, was durch die beiden Verknüpfungen 6.1 bzw. 7.1 verdeutlicht wird. Da es sich bei der Gaskomponente 7 um ein Leitgas handelt, genügt es auch hier, für die Erkennung des Geruchs 2 lediglich das Leitgas 7 zu erfassen. Verändert sich somit der Widerstand des Sensors 12, bedeutet dies eine Veränderung der Konzentration des Leitgases 7 und damit eine Veränderung in der Konzentration des Geruchs 2. Der Geruch 3 setzt sich nur aus dem Leitgas 8 zusammen, was durch die Verknüpfung 8.1 veranschaulicht wird. Eine Veränderung des Widerstands des Sensors 13 bedeutet somit eine Veränderung der Konzentration des Leitgases 8 und damit wiederum eine Veränderung der Konzentration des Geruchs 3.

Die Punktlinien zwischen der Gaskomponente 5 und der Gaskomponente 6 sowie zwischen der Gaskomponente 7 und der Gaskomponente 8 sollen andeuten, dass mit dem vorgeschlagenen Verfahren annähernd beliebig viele Gaskomponenten erfasst werden können. Das Verknüpfungsbild wäre dann um die entsprechenden Gerüche, Gaskomponenten und Sensoren zu erweitern.

Verknüpft werden können beispielsweise Gaskomponenten, die entweder selbst geruchsaktiv sind oder zwingend mit geruchsaktiven Gaskomponenten einhergehen, was für Vorläufer-, Begleit- und/oder Zerfallsprodukte der Fall ist.

Die Anzahl der für die Erfassung der Konzentration einer Gaskomponente erforderlichen Sensoren hängt von der Gaskomponente selbst und den zur Verfügung stehenden Sensoren ab. So kann es beispielsweise vorkommen, dass eine Gaskomponente nur dann mit ausreichender Sicherheit erfasst werden kann, wenn drei Sensoren verwendet werden, auch wenn dies in Figur 1 nicht dargestellt ist.

Am besten geeignet sind Sensoren, deren Querempfindlichkeit auf Gaskomponenten, die nicht erfasst werden sollen, so gering wie möglich ist.

Das Verfahren ist aufgrund der Selektivität der verwendeten Sensoren besonders robust gegen eine Empfindlichkeitsdrift, was bei Metalloxid-Sensoren häufig der Fall ist.

Die Erfassung von Art und Intensität des Geruchs ermöglicht es, mittels einer Steuerung die Umluftklappe automatisch zu steuern. Daher kann im Bedarfsfall, das heißt, wenn ein entsprechender Geruch mit ausreichender Intensität erfasst wurde, die Umluftklappe geschlossen werden und damit der Innenraum des Fahrzeugs von der Zufuhr von Frischluft getrennt werden. Damit ist der Insasse gegen die vom Sensor-System erfassten Gerüche geschützt.

Für das Sensor-System können verschiedene Prinzipien von Sensoren verwendet werden. Prinzipiell sind Sensoren aller Technologien verwendbar, mit denen die gewünschten Leitgase erfasst werden können. Als Sensoren kommen daher beispielsweise Adsorptionsspektroskopie-Sensoren, Oberflächenwellen-Sensoren, auch als Surface Acoustic Wave (SAW) Sensoren bekannt, oder Bulk Acoustic Wave Sensoren in Frage. Die Verwendung von Metalloxid-Sensoren oder Halbleitersensoren ist aus Kostengründen vorteilhaft.

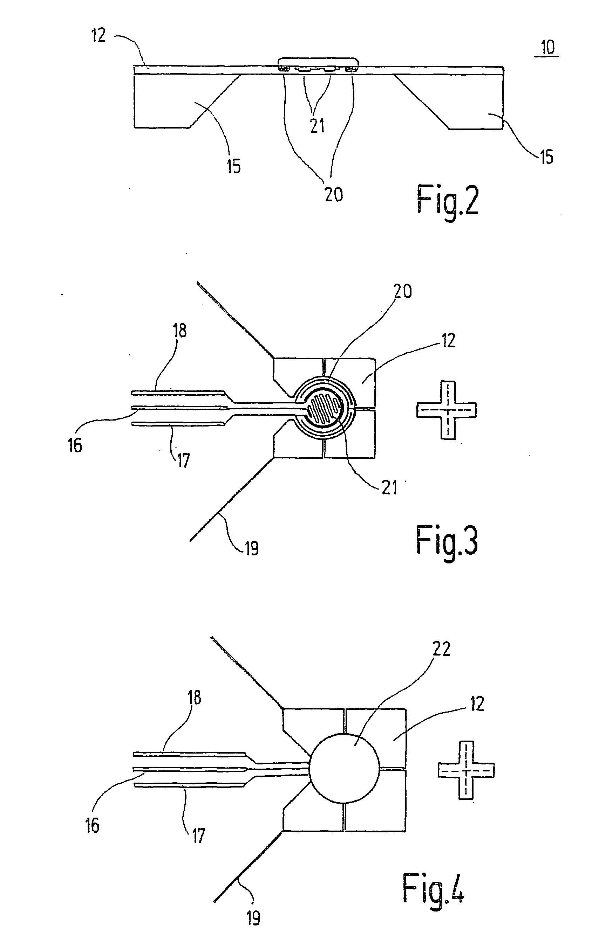

Im Folgenden wird anhand der Figuren 2, 3 und 4 der Aufbau eines Halbleitersensors näher erläutert. In Figur 2 ist beispielhaft der Aufbau des Sensorelements 10 aus Figur 1 im Querschnitt gezeigt. Die Sensoren 11, 12 und 13 können ebenfalls auf die in Figur 2 gezeigte Art und Weise aufgebaut sein.

Der Gassensor 10 umfasst ein Siliziumsubstrat 15, auf welchem eine Membran 12 angeordnet ist. In die Membran 12 ist eine Heizstruktur 20 sowie eine Elektrodenstruktur 21 eingebettet. Die Elektrodenstruktur 21 ist, wie in den Figuren 3 und 4 gezeigt, mit einer Zuleitung 16 und die Heizstruktur 20 mit zwei Zuleitungen 17 und 18 zum Anschluss an eine Spannungsquelle versehen.

Die Heizstruktur 20 und die Elektrodenstruktur 21 sind von einer sensitiven Schicht 22, welche in Figur 4 dargestellt ist, bedeckt. Die sensitive Schicht 22 weist pulverförmige Metalloxide auf, die mittels eines Brennprozesses versintert werden. Mit Hilfe der Heizstruktur 20 wird die sensitive Schicht 22 auf eine Temperatur in Höhe von 100 bis 400 Grad Celsius gebracht. Bei einer Anwesenheit der zu erfassenden Gase ändert sich der Widerstand der sensitiven Schicht 22, der mittels der Elektrodenstruktur 21 ausgewertet werden kann.

Die sensitive Schicht 22 weist Metalloxide der Metall Sn, Zn, Fe, Mo oder Co auf. Zudem sind 0,001-1% Cu, Ni, MoS2 beigemengt. Weiterhin können 0,001-1% Edelmetalle, wie beispielsweise Pt oder Pd, beigemengt sein.

Das Grundmaterial aus Metalloxid sowie die Beimengungen werden so gewählt, dass das Signal des Gassensors, bedingt durch die Änderung des Widerstands, für die jeweilige gewünschte Leitsubstanz maximal wird. Auf diese Art können verschiedene sensitive Schichten 22 für verschiedene Leitsubstanzen erzeugt werden.

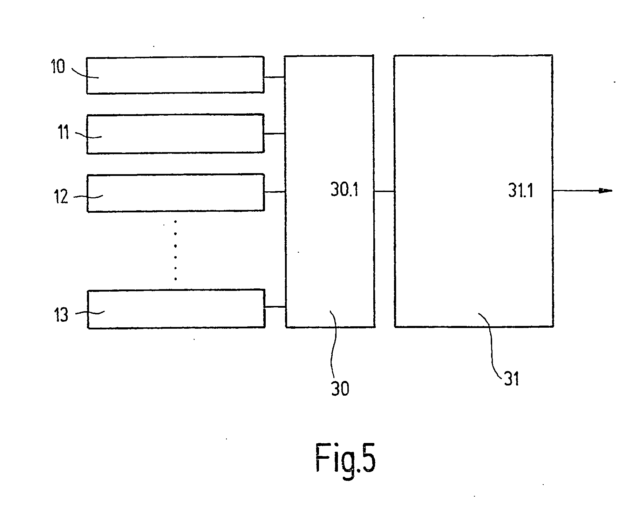

Ein Sensor-System, das für das erfindungsgemäße Verfahren zur Erfassung von Gerüchen geeignet ist, ist in Figur 5 in Form eines Blockdiagramms dargestellt. Eine Reihe von Sensoren 10, 11, 12 und 13, wobei jeder Sensor 10 bis 13 auf ein Leitgas spezialisiert ist, sind mit einem Interface 30 verbunden. Das Interface 30 bereitet die von den Sensoren 10 bis 13 erzeugten Sensorsignale, beispielsweise mit Hilfe einer Widerstands-Frequenz-Wandlung, auf und stellt an seinem Ausgang 30.1 das entsprechend aufbereitete Sensorsignal einem Mikrocontroller 31 zur Verfügung. Mit Hilfe des Mikrocontrollers 31 wird das oben beschriebene Verfahren abgearbeitet. Im in Figur 5 gezeigten Ausführungsbeispiel werden vier Sensoren 10 bis 13 verwendet. Die Leitgase sind Kohlenmonoxid CO, Stickoxid NO, und erfindungsgemäß Trans-2-Buten-1-thiol und Trans-2-Butenylthioacetat.

Über die genannten Leitsubstanzen ist eine Erkennung von Abgasen (CO) eines Otto-Motors, von Abgasen (NO) eines DieselMotors und von Skunksekreten (Trans-2-Buten-1-thiol) und Trans-2-Butenylthioacetat) mit ausreichender Genauigkeit möglich. Die Skunksekret-Erkennung benutzt dabei die Tatsache, dass die Erkennung von zwei Leitsubstanzen die komplexe Geruchssituation "Skunk" ausreichend kennzeichnet.

Am Ausgang 31.1 des Mikrocontrollers 31 steht ein Steuersignal zur Verfügung, mit dessen Hilfe die Umluftklappe eines Kraftfahrzeugs gesteuert werden kann.

## Patentansprüche

1. Verfahren zur Erfassung von Geruch, bei dem zuerst eine Zuordnung erfolgt, indem dem zu erfassenden Geruch zumindest eine entsprechende Gaskomponente und jeder zu erfassenden Gaskomponente aus einer Anzahl von Sensoren zumindest ein dafür geeigneter Sensor zugeordnet wird, und anschließend eine Auswertung erfolgt, indem von von den Sensoren erzeugten Sensorsignalen über die Zuordnung auf die Gaskomponenten und von den Gaskomponenten auf den Geruch geschlossen wird, **dadurch gekennzeichnet, dass** mittels des Verfahrens unterschiedliche Gerüche (1, 2, 3) erfassbar sind, wobei jedem der unterschiedlichen Gerüche (1, 2, 3) eine oder mehrere entsprechende Gaskomponenten (4, 5; 6, 7; 8) zugeordnet wird bzw. werden, und wobei mit den Sensoren (10, 11, 12, 13) Trans-2-Buten-1-thiol und/oder Trans-2-Butenylthioacetat erfasst werden.

2. Verfahren nach Patentanspruch 1, **dadurch gekennzeichnet, dass** mittels jeweils einem Sensor (10, 11, 12, 13) jeweils ein Gas erfasst wird.

3. Verfahren nach Patentanspruch 1 oder 2, **dadurch gekennzeichnet, dass** die Sensoren (10, 11, 12, 13) als Halbleiter-Gassensoren ausgebildet sind.

4. Verfahren nach einem der Patentansprüche 1 bis 3, **dadurch gekennzeichnet, dass** das Sensorsignal vom Widerstand des Sensors (10, 11, 12, 13) abhängt.

5. Verfahren nach Patentanspruch 1 oder 2, **dadurch gekennzeichnet, dass** die Sensoren (10 11, 12, 13) als Adsorptionsspektroskopie-Sensoren ausgebildet sind.

6. Verfahren nach Patentanspruch 1 oder 2, **dadurch gekennzeichnet, dass** die Sensoren (10, 11, 12, 13) als Oberflächenwellen-Sensoren ausgebildet sind.

7. Verfahren nach Patentanspruch 1 oder 2, **dadurch gekennzeichnet, dass** die Sensoren (10, 11, 12, 13) als Bulk Acoustic Wave Sensoren ausgebildet sind.

8. Verfahren nach einem der Ansprüche 1 bis 7, **dadurch gekennzeichnet, dass** anhand der erfassten Gerüche (1, 2, 3) eine Umluftklappe eines Kraftfahrzeugs gesteuert wird.

9. Verfahren nach einem der Patentansprüche 1 bis 8, **dadurch gekennzeichnet, dass** mit den Sensoren (10, 11, 12, 13) CO und/oder NO erfasst werden.

10. Verfahren nach einem der Patentansprüche 1 bis 9, **dadurch gekennzeichnet, dass** die Gaskomponenten geruchsaktive Gase oder Gase sind, die mit geruchsaktiven Gasen einhergehen.

## Claims

1. A method for detecting odour in which an assignment is firstly made whereby at least one corresponding gas component is assigned to the odour to be detected and at least one sensor suitable for this purpose is assigned from a number of sensors to each gas component to be detected and then an evaluation is made, whereby the odour is inferred from sensor signals generated by the sensors by means of the assignment to the gas components and from the gas components, **characterised in that** different odours (1, 2, 3) can be detected by means of the method, wherein one or more corresponding gas components (4, 5; 6, 7, 8) is or are assigned to each of the different odours (1, 2, 3) and wherein trans-2-butene-1-thiol and/or trans-2-butenyl thioacetate are detected with the sensors (10, 11, 12, 13).

2. The method according to claim 1, **characterised in that** respectively one gas is detected by means of respectively one sensor (10, 11, 12, 13).

3. The method according to claim 1 or 2, **characterised in that** the sensors (10, 11, 12, 13) are configured as semiconductor gas sensors.

4. The method according to one of claims 1 to 3, **characterised in that** the sensor signal depends on the resistance of the sensors (10, 11, 12, 13).

5. The method according to claim 1 or 2, **characterised in that** the sensors (10, 11, 12, 13) are configured as adsorption spectroscopy sensors.

6. The method according to claim 1 or 2, **characterised in that** the sensors (10, 11, 12, 13) are configured as surface wave sensors.

7. The method according to claim 1 or 2, **characterised in that** the sensors (10, 11, 12, 13) are configured as bulk acoustic wave sensors.

8. The method according to one of claims 1 to 7, **characterised in that** an air admission flap of a motor vehicle is controlled by means of the detected odours (1, 2, 3).

9. The method according to one of claims 1 to 8, **characterised in that** CO and/or NO are detected with the sensors (10, 11, 12, 13).

10. The method according to one of claims 1 to 9, **characterised in that** the gas components are odour-active gases or gases which are associated with odour-active gases.

## Revendications

1. Procédé de détection d'odeurs, dans lequel une affectation a tout d'abord lieu, en ce que au moins un composant de gaz correspondant est affecté à l'odeur à détecter et au moins un capteur approprié à ces fins d'une pluralité de capteurs est affecté à chacun des composants de gaz à détecter, et une évaluation a ensuite lieu, en ce que l'odeur est déterminée à partir des signaux de capteurs générés par les capteurs via l'affectation aux composants de gaz et à partir des composants de gaz, **caractérisé en ce que** des odeurs différentes (1,2,3) peuvent être détectées au moyen du procédé, moyennant quoi un ou plusieurs composants de gaz (4,5 ;6,7) correspondants est, respectivement sont affectés à chacune des odeurs différentes (1,2,3) et moyennant quoi les capteurs (10,11,12,13) détectent du trans- 2- butène -1 -thiol et/ou du trans-2-buténylethioacétate.

2. Procédé selon la revendication 1, **caractérisé en ce que** un gaz respectif est détecté au moyen d'un capteur (10,11,12,13) respectif.

3. Procédé selon la revendication 1 ou 2, **caractérisé en ce que** les capteurs (10,11,12,13) sont réalisés comme des capteurs de gaz à semi-conducteurs.

4. Procédé selon une des revendications 1 à 3, **caractérisé en ce que** le signal de capteur dépend de la résistance du capteur (10,11,12,13).

5. Procédé selon la revendication 1 ou 2, **caractérisé en ce que** les capteurs (10,11,12,13) sont réalisés comme des capteurs de spectroscopie d'adsorption.

6. Procédé selon la revendication 1 ou 2, **caractérisé en ce que** les capteurs (10,11,12,13) sont réalisés comme des capteurs d'ondes de surface.

7. Procédé selon la revendication 1 ou 2, **caractérisé en ce que** les capteurs (10,11,12,13) sont réalisés comme des capteurs d'ondes acoustiques de volume.

8. Procédé selon une des revendications 1 à 7, **caractérisé en ce que**, sur la base des odeurs (1,2,3) détectées, un volet de recirculation d'air d'un véhicule automobile est commandé.

9. Procédé selon une des revendications 1 à 8, **caractérisé en ce que** les capteurs (10,11,12,13) détectent du CO et/ou du NO.

10. Procédé selon une des revendications 1 à 9, **caractérisé en ce que** les composants de gaz sont des gaz odorants ou des gaz, qui s'accompagnent de gaz odorants.
